# EUROPÄISCHE PATENTSCHRIFT

(11) **EP 2 931 345 B1**
(45) Veröffentlichungstag und Bekanntmachung des Hinweises auf die Patenterteilung: **12.04.2017**
(21) Anmeldenummer: 13815399.4
(22) Anmeldetag: 05.12.2013
(51) Int. Cl.: A61M 16/04, A61M 25/10

(54) **DRUCKAUSGLEICHSBALLON UND VERFAHREN ZU DESSEN HERSTELLUNG**
PRESSURE EQUALIZATION BALLOON AND METHOD FOR THE PRODUCTION THEREOF
BALLONNET DE COMPENSATION DE PRESSION ET SON PROCÉDÉ DE FABRICATION

(30) Priorität: 11.12.2012 DE 102012112095
(43) Veröffentlichungstag der Anmeldung: 21.10.2015
(73) Patentinhaber: TRACOE medical GmbH, 55268 Nieder-Olm (DE)
(72) Erfinder: SCHNELL, Ralf, 63500 Seligenstadt (DE); HAHN, Andreas, 55291 Saulheim (DE)
(74) Vertreter: WSL Patentanwälte Partnerschaft mbB
(86) Internationale Anmeldenummer: PCT/EP2013/075657
(87) Internationale Veröffentlichungsnummer: WO 2014/090679

(56) Entgegenhaltungen:
- EP-A2- 1 252 909
- WO-A1-91/10465
- WO-A1-2008/038430
- DE-A1- 3 433 785
- US-A- 3 642 005
- US-A- 4 018 231
- US-A- 4 119 101
- US-A- 4 552 558
- US-A- 4 649 914
- G. P. Hellmann: "Ursachen von Restdehnung und Energieverlust bei TPE", , 1 December 2000 (2000-12-01), XP055285701, Retrieved from the Internet: URL:http://www.fgkunststoffe.de/sites/defa ult/files/8098.pdf [retrieved on 2016-07-04]

## Beschreibung

Die vorliegende Erfindung betrifft einen Druckausgleichsballon, wobei das Volumen des Druckausgleichsballons mit einem weiteren Volumen in Verbindung steht, dessen Druck auch bei einer aufgezwungenen Volumenänderung einen möglichst konstanten Wert behalten soll.

Ein solches Volumen, dessen Druck auch bei einer von außen aufgezogenen Volumenänderung einen möglichst konstanten Wert behalten soll, ist beispielsweise das Volumen der Manschette einer Beamtungskanüle. Die Manschette, in der medizinischen Fachsprache und im Folgenden auch "Cuff" genannt, umgibt dabei eine in die Trachea (Luftröhre) eines Patienten eingeführte Kanüle und wird nach dem Einführen der Kanüle auf einen geringen Druck in der Größenordnung von 20 bis 30 mbar aufgeblasen, damit sie sich dichtend an die Wand der Trachea anlegt, die Kanüle auf diese Weise fixiert und außerdem verhindert, das Sekret, welches sich oberhalb des Cuffs in der Umgebung der Beatmungskanüle ansammelt, in die Bronchien bzw. in die Lunge des Patienten gelangt. Ansonsten könnte derartiges Sekret, in welchem sich Krankheitskeime sehr schnell vermehren und welches dann in die tieferen Atemwege oder die Lunge gelangt, zu schweren Komplikationen wie z. B. einer Lungenentzündung führen.

Eine gute Abdichtung durch einen solchen Cuff ist insbesondere bei langzeitbeatmeten Patienten von großer Bedeutung. Die Kanüle kann dabei entweder ein Endotrachealtubus sein, der durch Mund und Rachen eines Patienten eingeführt wird, kann aber ebenso gut auch eine Tracheostomiekanüle sein, die durch eine chirurgisch erzeugte Öffnung (ein Tracheostoma) im Hals eines Patienten hindurchgeführt ist und damit unter Umgehung des Mund- und Rachenraums die Trachea mit der Umgebungsluft oder einem Beatmungsgerät verbindet.

Insbesondere bei langzeitbeatmeten Patienten ist dabei auch der eingestellte Cuff-Druck von größter Bedeutung, da dieser Druck auf Dauer einen Wert von 30 mbar nicht übersteigen darf, weil der auf die sehr empfindliche Wand der Trachea wirkende Druck zu einer Behinderung der Durchblutung und im Ergebnis zu Beschädigungen bzw. Wunden bis hin zu Nekrosen führen kann.

Andererseits darf der Cuff-Druck auch nicht zu gering sein, um den Vorbeitritt des oben erwähnten Sekretes an dem Cuff vorbei zu verhindern. Außerdem könnte unkontrolliert an dem Cuff vorbeiströmende Luft auch einen Fehlalarm an einem Beatmungsgerät auslösen, da die Beatmung bei Verwendung entsprechender Kanülen grundsätzlich über das Lumen der Kanüle erfolgen soll und anhand des Luftstroms durch die Kanüle kontrolliert wird.

Um den Cuff-Druck daher auf dem im Allgemeinen als sinnvoll und optimal angesehen Druckbereich zwischen etwa 20 mbar und 30 mbar zu halten, sind bereits zahlreiche Einrichtungen verwendet und beschrieben worden, die den Druck in dem Cuff konstant halten sollen. Dabei ist zu berücksichtigen, dass z. B. durch Verschieben, Verdrehen oder Neigen des Kopfes eines Patienten auch der Tracheaquerschnitt sich ändern kann und damit in unterschiedlicher Weise auf den Cuff drückt und dessen Volumen verändert. Der Cuff muss daher über einen entsprechenden Schlauch, der im Allgemeinen an der Kanüle entlang geführt oder in die Wand der Kanüle integriert ist, mit einem entsprechenden Druckreservoir verbunden sein, welches je nach Volumenänderung des Cuffs Luft bzw. generell Füllgas wie z. B. Stickstoff, aus dem Cuff aufnehmen kann und auch wieder in den Cuff zurückführt, um den Cuff-Druck unabhängig von einem möglicherweise variierenden Cuffvolumen auf einem konstanten Wert zu halten.

Hierzu sind beispielsweise elektronische Druckregler bekannt ebenso wie auch einfache Ausgleichsvolumina, insbesondere Ballons, wobei in aller Regel der Druck eines Cuffs von medizinischem Fachpersonal in gewissen zeitlichen Abständen, oftmals jedoch nur einmal pro Schicht, kontrolliert und nachjustiert wird.

Des Weiteren ist auch bereits ein System bekannt, welches mit einem Latexballon als Ausgleichsvolumen arbeitet, wobei der Latexballon die Eigenschaft hat, zumindest innerhalb eines gewissen Volumenbereichs einen konstanten Druck aufrecht zu erhalten. Das heißt, bereits eine geringfügige Erhöhung des Drucks führt zu einer entsprechenden Volumenzunahme des Latex-ballons, während eine geringe Druckabnahme wieder zu einer Volumenverringerung des Latexballons führt, so dass im Ergebnis der Druck in einem Cuff, der mit einem entsprechenden Latexballon verbunden ist, auf demselben in etwa konstanten Wert bleibt. Um den sehr dünnen und empfindlichen Latexballon zu schützen, der die gewünschten Eigenschaften hat, ist er in einer äußeren Schutzhülle angeordnet, die vorzugsweise durchsichtig ist, im Wesentlichen Kugelform hat und stabil genug ist, um den in der Schutzhülle aufgenommenen Ballon gegenüber Stößen, Beschädigungen oder von außen ausgeübtem Druck zu schützen und es versteht sich, dass die Schutzhülle luftdurchlässig sein muss, um die Ausdehnung oder ein Zusammenziehen des Ausgleichsballons innerhalb der Schutzhülle nicht zu beeinflussen.

Verschiedene Vorrichtungen zur Konstanthaltung oder Überwachung des Drucks entsprechender Cuffs oder Manschetten im medizinischen Bereich sind bekannt aus den US-Patenten 3,642,005, 4,501,273, der US-Patentanmeldung 2012/0090619 A1, der EP 1 252 909 A2, der US 4,018,231 A und dem britischen Patent GB 1 423 789.

Elektronische Druckregelsysteme sind sehr aufwendig und teuer. Ballonsysteme mit sehr voluminösen Ausgleichsballons sind unhandlich und unterliegen der Gefahr von Beschädigungen eines solchen Ballons. Das bekannte System mit einem Ausgleichsballon aus Latex, der sich innerhalb einer stabilen, durchsichtigen Schutzhülle befindet, funktioniert im Prinzip relativ gut, hat aber den Nachteil, dass das Latex u. a. aufgrund seines allergenen Potentials in vielen Bereichen der medizinischen Behandlung unerwünscht oder gar unzulässig ist.

Die EP 1 252 909 A2 beschreibt bereits ein System aus einem Hauptballon (entsprechend dem Cuff) und einem Pilotballon, wobei Hauptballon und Pilotballon dieselbe Elastizität aufweisen sollen, so dass der Zustand oder auch insbesondere der Druck des Hauptballons (Cuff) anhand des Zustandes des sichtbaren Pilotballons ablesbar ist. Die EP 1 252 909 beschreibt dabei sowohl für den Hauptballon als auch für den Pilotballon ein Material aus thermoplastischen Elastomeren auf Styrolbasis.

Gegenüber diesem Stand der Technik liegt der vorliegenden Erfindung die Aufgabe zugrunde, einen Druckausgleichsballon bereitzustellen, welcher einen vorgegebenen Druck in einem anderen, an den Ausgleichsballon angeschlossenen Volumen, wie zum Beispiel einem Cuff bzw. einer Manschette, im Wesentlichen konstant hält, ohne dabei die Nachteile des vorgenannten Standes der Technik aufzuweisen.

Diese Aufgabe wird dadurch gelöst, dass der Ausgleichsballon aus einem thermoplastischen Polymer (TPE) hergestellt ist, und der Ballon vor dem ersten Gebrauch über das vorgesehene maximale Regelvolumen hinaus überdehnt und anschließend wieder relaxiert wurde, wobei das Überdehnungsvolumen mindestens das Doppelte des maximalen Regelvolumens beträgt, wodurch der Elastizitätsmodul des TPE sich in Abhängigkeit von der Dehnung in der Weise ändert, dass der in dem Ausgleichsballon herrschende Druck mindestens innerhalb eines vorgegebenen Regelvolumenbereichs des Ausgleichsballons konstant bleibt, indem das Volumen des Ausgleichsballons bei einem über einen gewünschten Regelwert steigenden Druck zunimmt und bei einem unter den gewünschten Regelwert abnehmenden Druck abnimmt, wobei das regelvolumen im Bereich von 20 cm³ bis 100 cm³ liegt.

Der vorliegenden Erfindung liegt die Erkenntnis zugrunde, dass Ballons, die aus einem thermo-plastischen Polymer hergestellt sind, in der Regel zwar eine mehr oder weniger lineare Zunahme des Drucks mit dem Volumen zeigen, wie sich dies auch aus der EP 1 252 909 indirekt ergibt, dass es jedoch möglich ist, die elastischen Eigenschaften von thermoplastischen Polymeren durch eine kontrollierte Überdehnung gezielt zu beeinflussen. Konkret erfolgt die Überdehnung auf ein Volumen des Ausgleichsballons, welches deutlich oberhalb des Volumens liegt, welches für den Regelbetrieb des Ausgleichsballons vorgesehen ist. Dies führt innerhalb des Regelvolumenbereichs zu einem Dehnungsverhalten des Materials, dass nur eine äußerst geringe Druckabhängigkeit aufweist, so dass (jedenfalls innerhalb des Regelvolumenbereichs) der Druck innerhalb des Ausgleichsballons (und damit auch in jedem damit verbundenen Volumen) im Wesentlichen konstant bleibt. Der Regelvolumenbereich ist dabei praktisch der gesamte Bereich des Volumens des Ausgleichsballons vom drucklosen Zustand (nach dem Überdehnen) und einem Wert, der typischerweise zwischen dem Zwei- und Zehnfachen des Volumens im drucklosen Zustand liegt.

Zweckmäßigerweise wird ein entsprechender Ausgleichsballon für die Verwendung an Beatmungskanülen mit einem Anfangsvolumen (vor dem Überdehnen) von etwa 3 bis 8 cm³ hergestellt, welches sich nach dem erstmaligen Überdehnen, z. B. auf ein Volumen von etwa 200 cm³ (oder auch mehr), um etwa 20 bis 50% vergrößert hat. Das Regelvolumen könnte dann im Bereich von 20 bis 100 cm³ liegen, vorzugsweise im Bereich von 30 bis 70 cm³.

Der Ausgleichsballon ist vorzugsweise von einer Schutzhülle umgeben, die den relativ empfindlichen Ausgleichsballon vor äußeren mechanischen Einwirkungen schützt. Eine solche Schutzhülle, z. B. aus stabilem, durchsichtigen Kunststoffmaterial und zweckmäßigerweise im Wesentlichen in Kugelform sollte dann einen Durchmesser in der Größenordnung von 50 bis 60 mm haben.

Ein besonderer Vorteil der Verwendung von thermoplastischen Polymeren liegt darin, dass ein entsprechender Ballon im Spritzgussverfahren hergestellt werden kann, was eine sehr gut kontrollierbare, gleichmäßige Wandstärke ermöglicht, wobei die Wandstärke wiederum einer der Parameter ist, durch welche der konkrete Regeldruck eingestellt werden kann. Ein zweiter Parameter für die Einstellung eines Regeldrucks liegt in dem Ausmaß der Überdehnung.

Typischerweise beginnt der Bereich der Überdehnung etwa bei dem zehn- bis zwanzigfachen des Ausgangsvolumens im drucklosen Zustand nach dem Spritzgießen, kann aber auch ohne Weiteres bis zum Hundertfachen des Ausgangsdrucks betragen.

Eine stärkere Überdehnung führt dabei zu einer Verminderung des Regeldrucks (in dem selben Regelvolumenbereich), gegenüber demjenigen Regeldruck, den man bei einer geringeren Überdehnung erhalten würde. Der Regeldruck muss dabei kein exakter Punktwert sein, denn eine geringfügige Volumenabhängigkeit des Druckes, beispielsweise eine Variation von 1 mbar über den Regelvolumenbereich hinweg, ist für praktische Zwecke als konstanter Druck anzusehen. Aufgrund der variabel einzustellenden Überdehnung ist es möglich, beim Spritzgießen eine Wandstärke des Ballons zu wählen, die für die praktische Handhabung gut geeignet ist und nicht allzu leicht zu einer Beschädigung des empfindlichen Ballonmaterials führt. Eine größere Wandstärke kann dabei in relativ einfacher Weise durch eine stärkere Überdehnung ausgeglichen werden, um dennoch zu dem selben Regeldruck innerhalb eines gewissen Volumenbereichs zu gelangen.

Als besonders geeignet haben sich styrolbasierte thermoplastische Polymere erwiesen, wie z. B. SBS (Styrol-Butadien-Styrol), SIS (Styrol-Isopren-Styrol) und SEPS (Styrol-Ethylen-Propylen-Styrol). Besonders bevorzugt ist für die vorliegende Erfindung das Material SEBS (Styrol-Ethylen-Butylen-Styrol).

Das thermoplastische Material kann auch Füllstoffe, wie zum Beispiel Mineralöle und insbesondere medizinische Weißöle aufweisen.

Zweckmäßigerweise wird das thermoplastische Material so ausgewählt bzw. eingestellt, dass es eine Shore 00 Härte (nach ISO 868) von 30 - 70, vorzugsweise 50 Shore 00 hat. Der Zugmodul bei 100 % Dehnung liegt typischerweise im Bereich zwischen 40-150 kPa (nach ISO 37).

Die Wandstärke des drucklosen Ballons vor dem erstmaligen Überdehnen, d. h. nach dem Spritzgießen, kann z. B. im Bereich zwischen 0,4 und 1,5 mm liegen. Beim Dehnen eines solchen Ballons und insbesondere zum Überdehnen wird typischerweise ein Druck in der Größenordnung von 40 bis 60 mbar benötigt. Lässt man jedoch den Ballon nach einem hinreichenden Überdehnen wieder relaxieren und zurück in den drucklosen Zustand gelangen, so hat er in diesem Zustand zum einen ein im Allgemeinen etwas größeres Volumen als nach dem Spritzgießen, vor allem aber ein anderes Dehnungsverhalten, d. h. der Druck, der zum Aufblähen des Ballons innerhalb des Regelvolumenbereichs (typischerweise weniger als ein Viertel des Überdehnungsvolumens) benötigt wird, liegt jedoch nur noch bei etwa der Hälfte des Druckes, der für das erstmalige Dehnen und Überdehnen des Ballons erforderlich ist und er bleibt über einen größeren Volumenbereich des Ballons hinweg weitgehend konstant. Wie gesagt kann der genaue Wert durch gezielte Wahl der Wandstärke und durch das kontrollierte Ausmaß der Überdehnung relativ genau eingestellt werden.

Zwar zeigt das Material gelegentlich einen gewissen "Erholungseffekt", wenn es längere Zeit im drucklosen Zustand gehalten wurde, was dazu führt, dass beim erstmaligen Wiederaufblähen der Regeldruckwert etwas überschritten werden muss; wird jedoch das zur Verfügung stehende Regelvolumen (welches beispielsweise durch die Schutzhülle begrenzt wird) vor der in Betriebnahme noch einmal vollständig ausgenutzt, so stellt sich das gewünschte Dehnungsverhalten bei dem Regeldruck sehr schnell wieder ein.

Ein weiterer Vorteil der Herstellung mittels Spritzgussverfahren ist es, dass der Ausgleichsballon an verschiedenen Stellen wahlweise mit unterschiedlichen Wandstärken hergestellt werden kann. Insbesondere ist in einer bevorzugten Ausführungsform der Erfindung vorgesehen, dass der Ausgleichsballon eine Ballonöffnung umgebenden verstärkten Öffnungsring aufweist, dessen Wandstärke mindestens 50% größer ist als die Wandstärke des Ballons im übrigen und der dementsprechend an der Dehnung beim Druckausgleich nicht teilnimmt. Ein solcher Öffnungsring kann mit einer genau definierten Geometrie und Wandstärke hergestellt werden, so dass er beispielsweise unmittelbar auf einen entsprechenden Standardanschluss (z. B. Luuer-Connector) in dichtem Eingriff aufgesetzt werden kann.

Der Öffnungsring kann insbesondere auch einen umlaufenden Dichtungsflansch aufweisen, der an einem entsprechenden Anschlussstutzen der Verbindungsleitung zu einem Cuff leicht fixiert werden kann.

Entsprechende Standardanschlüsse haben typischerweise einen Durchmesser im Bereich von 12 bis 25 mm.

Der Ballon sollte zweckmäßigerweise so hergestellt werden, dass das nutzbare Regelvolumen den Bereich von 10 bis 100 cm³, zweckmäßigerweise den Bereich von 20 bis 80 cm³ umfasst. Das maximale Regelvolumen wird zweckmäßigerweise begrenzt durch eine stabile, äußere Schutzhülle in Kugelform. Dadurch ist der Ballon im Inneren der durchsichtigen Hülle sichtbar und das Fachpersonal, welches die entsprechenden Beatmungsvorrichtungen bedient, kann anhand des Zustandes des Ballons unmittelbar erkennen, ob der Druck der Manschette bzw. des Cuffs im gewünschten Bereich liegt.

Wie bereits erwähnt, könnte eine entsprechende kugelförmige Schutzhülle aus Plexiglas oder irgendeinem anderen geeigneten, stabilen Kunststoffmaterial einen Durchmesser in der Größenordnung von 50 bis 60 mm und damit ein Volumen in der Größenordnung von 70 bis 100 cm³ haben. Die Schutzhülle kann eventuell auch kleiner gehalten werden und einen Durchmesser von 40 mm und bis herab zu 30 mm oder weniger aufweisen. Es versteht sich, dass die entsprechende Schutzhülle mindestens eine Öffnung aufweist, die das Innere der Schutzhülle mit der Umgebung verbindet, um außerhalb des Ausgleichsballons immer den konstanten Umgebungsdruck aufrecht zu erhalten, so dass der Ausgleichsballon sich innerhalb des Volumens der Schutzhülle ungehindert ausdehnen und zusammenziehen kann.

In einer Ausführungsform könnte der Ballon auch abwechselnde, vorzugsweise streifenförmige Bereiche größerer und kleinerer Wandstärke aufweisen. Dies würde es beispielsweise ermöglichen, gezielt eine gewisse Druckabhängigkeit des Volumens bereitzustellen, die es ermöglichen würde, den gezielt vorgesehenen Regeldruck zumindest zeitweise und im kontrollierten Umfang zu überschreiten. Dies ist beispielsweise in manchen Notsituationen erforderlich, wenn z. B. Blutungen in der Trachea bzw. im Hals- und Rachenraum auftreten und gestillt werden müssen, ohne dass die dichte Anlage des Cuffs an der Tracheawand aufgegeben wird, sondern ggf. sogar verstärkt werden muss.

In einer anderen Ausführungsform ist vorgesehen, dass die Schutzhülle, ausgehend von Ihrer kugelförmigen Grundform, einige, vorzugsweise streifenartig angeordnete, Ausbuchtungen aufweist. Dies führt beispielsweise dazu, dass der Ausgleichsballon sich bei einem geringfügigen Druckanstieg zunächst ausdehnt, bis er an Teilen der Innenwand der Schutzhülle anliegt, während danach eine weitere Dehnung des Ausgleichsballons nur noch im Bereich der Ausbuchtungen möglich ist. Dies bewirkt einen geringen aber deutlich spürbaren und sichtbaren Druckanstieg, bis auch die Ausbuchtungen mit dem Ballon ausgefüllt sind, woraufhin der Druck bei weiterer Gaszufuhr steil ansteigt. In dem Übergangsbereich, in welchem der Ballon sich in die Ausbuchtung hinein ausdehnt, ist jedoch ein verhältnismäßig leichter, kontinuierlicher Druckanstieg festzustellen, der es ermöglicht, gezielt und kontrolliert einen zeitweise gewünschten, höheren Cuff-Druck einzustellen, um beispielsweise eine Blutung in der Trachea im Bereich des Cuffs zu stillen.

In Analogie zu der Definition des Ballons selbst ist ein Verfahren zur Herstellung eines solchen Ausgleichsballons dadurch gekennzeichnet, dass der Ballon aus einem thermoplastischen Polymer, vorzugsweise aus SEBS, im Spritzgussverfahren hergestellt und vor dem ersten Gebrauch auf ein Volumen überdehnt wird, welches mindestens doppelt so groß wie das im Gebrauch vorgesehene maximale Regelvolumen ist.

Vorzugsweise beträgt das Überdehnungsvolumen mindestens das Zehnfache, vorzugsweise mindestens das Zwanzigfache des Volumens des Ausgleichsballons im drucklosen Zustand nach dem Spritzgießen.

Weiterhin ist das erfindungsgemäße Verfahren dadurch gekennzeichnet, dass eine Öffnung des Ballons mit einem die Öffnung umgebenden Öffnungsring größerer Wandstärke und definierter Geometrie für die Verbindung mit einem medizinischen Standardanschluss hergestellt wird. Weitere Vorteile, Merkmale und Anwendungsmöglichkeiten der vorliegenden Erfindung ergeben sich aus der folgenden Beschreibung einer bevorzugten Ausführungsform und der dazugehörigen Figuren.

Es zeigen:
- Figur 1: im linken Teilbild eine perspektivische Ansicht und im rechten Teilbild eine vertikale Schnittansicht durch einen erfindungsgemäßen Ausgleichsballon im drucklosen Zustand, wie er zum Beispiel unmittelbar nach der Herstellung im Spritzgussverfahren vorliegt,
- Figur 2: einen Druckausgleichsballon im aufgeblähten Zustand wie er während der Druckregelung vorliegt, wobei die Kontur des drucklosen Ballons ebenfalls wiedergegebene ist,
- Figur 3: einen Querschnitt durch den Ballon gemäß Figur 2 entsprechend der Linie 111-111 mit einer zusätzlich um den Ballon angeordneten Schutzhülle,
- Figur 4: eine schematische Ansicht auf eine weitere Ausführungsform der vorliegenden Erfindung,
- Figur 5:: eine Schnittansicht der Ausführungsform nach Figur 4 entsprechend der Schnittlinie V-V in Figur 4 und
- Figur 6:: eine Schnittansicht ähnlich der Figur 5 einer noch weiter abgewandelten Ausführungsform.

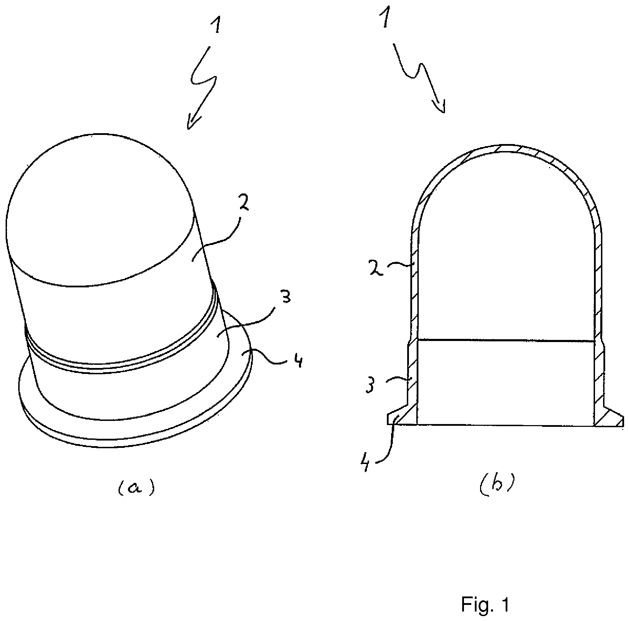

In Figur 1 erkennt man einen Druckausgleichsballon 1, der aus einem kuppelförmigen Dehnungsabschnitt 2, einem zylindrischen Abschnitt 3 mit etwas größerer Wandstärke und schließlich einem Flanschabschnitt 4 besteht, wobei die Abschnitte 3 und 4 einen Öffnungs- und Anschluss-Abschnitt des Druckausgleichsballons 1 bilden. Beispielsweise können die Abschnitte 3 und 4 auf einen im medizinischen Bereich typischen Luuer-Konnektor, d.h. einen Rohrstutzen, der einen passenden Durchmesser von z. B. 12 - 25 mm hat, aufgesteckt werden, wobei der freie Innendurchmesser der Abschnitte 3 und 4 vorzugsweise etwas kleiner ist als der Außendurchmesser des Rohrstutzens, auf welchen dieser Anschlussabschnitt aufgesteckt wird, damit er unter leichter elastischer Spannung dicht an der Außenseite des Rohrstutzens anliegt. Um den Abschnitt 3 herum kann auch noch ein Spannring oder Spannband angeordnet werden, der den Ballon sicher an dem Rohrstutzen festhält, wobei der Flanschabschnitt 4 als Sicherung dient.

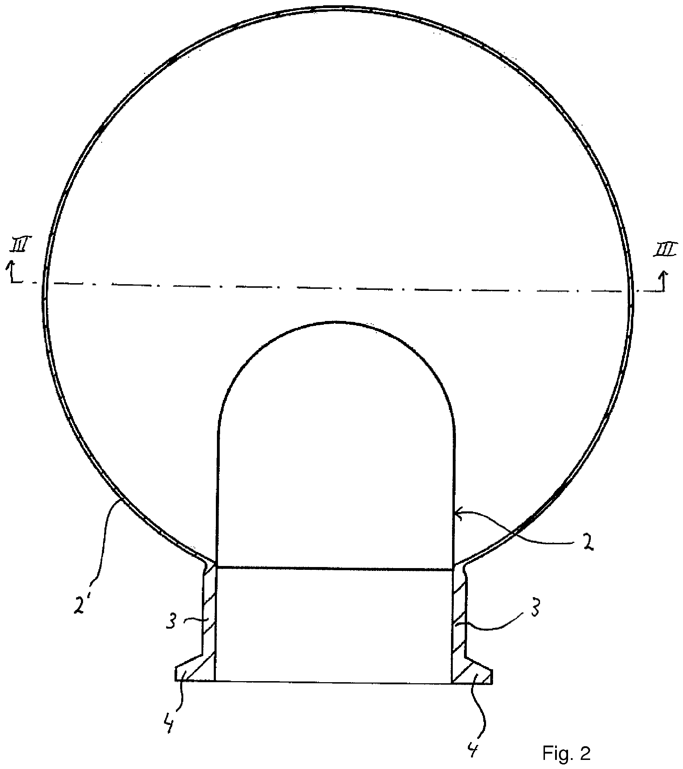

Figur 2 zeigt den Dehnungsabschnitt 2 in einem gedehnten Zustand, wobei der Dehnungsabschnitt im gedehnten Zustand mit 2' bezeichnet ist. Gleichzeitig ist auch die Kontur des ungedehnten Abschnittes 2 eingezeichnet. Die Größenverhältnisse zwischen dem Dehnungsabschnitt 2' im gedehnten und im drucklosen Zustand 2 gibt in etwa realistisch das Größenverhältnis in einem typischen Regelbereich wieder, in welchem der Druck im Inneren des Ausgleichsballons 2 unabhängig vom Volumen im Wesentlichen konstant bleibt. Mit anderen Worten, eine sehr geringfügige Erhöhung des Drucks, konkret die zufuhr eines Füllgases, führt unmittelbar zu einer entsprechenden Dehnung des Abschnittes 2', die den Druck wieder auf den Regelwert absenkt. Umgekehrt führt eine geringfügige Drucksenkung bzw. eine Abziehen oder Ablassen sofort zu einer Reduzierung des Volumens des Abschnittes 2', sodass auch dann wieder der Regeldruck erreicht wird.

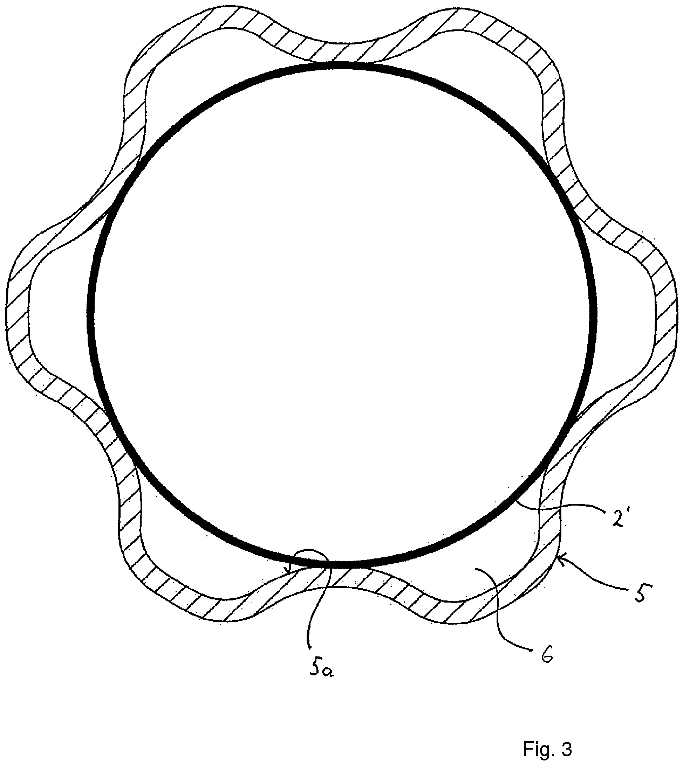

Figur 3 zeigt eine besondere Ausführungsform der vorliegenden Erfindung mit einer Schutzhülle 5, die aus einem relativ festen und im Vergleich zu dem Druckausgleichsballon 1 praktisch nicht dehnbaren Material besteht, welches vorzugsweise durchsichtig ist, sodass man den konkreten Zustand des Druckausgleichsballons 1 durch die Schutzhülle 5 hindurch sehen kann.

Im Allgemeinen könnte die Schutzhülle 5 kugelförmig sein mit einem Öffnungsabschnitt, welcher dem Anschlussabschnitt 3, 4 des Druckausgleichsballons 1 entspricht, sodass sich der Dehnungsabschnitt 2' bei Erreichen eines Radius, welcher dem Innenradius der Schutzhülle entspricht, vollflächig an die Innenwand der Schutzhülle anlegen würde, sodass im Falle einer weiteren Gaszufuhr in das Innere des Ballons der Druck sofort und im Wesentlichen proportional zur Zunahme der Gasmenge ansteigen würde.

Die Schutzhülle 5 gemäß Figur 3 weicht jedoch von einer Kugelform dadurch ab, dass sie von dem unteren Öffnungsbereich ausgehende, streifenförmige Ausbuchtungen 6 aufweist, wie man im Schnittbild der Figur 3 sehr deutlich erkennen kann, wohingegen ein Schnitt senkrecht zur Ebene der Figur 3 noch im Wesentlichen eine Kugelform bzw. von der Innenseite her rein konkave Form der Schutzhülle 5 wiedergeben würde. Diese Ausgestaltung führt dazu, dass der Dehnungsabschnitt 2' nach Erreichen eines Radius, welcher dem Radius der konvexen Abschnitte der Schutzhülle 5 entspricht, sich an die Innenwand 5a dieser konvexen Abschnitte anlegt, die in diesem Bereich eine weitere Dehnung des Abschnittes 2' nicht mehr zulassen.

Bei weiterer Gaszufuhr bzw. Erhöhung des Druckes muss sich der Dehnungsabschnitt daher in die Ausbuchtungen 6 hinein ausdehnen. Diese Einschränkung der Dehnung des Abschnittes 2' entlang der Wände 5a der konvexen Bereiche der Schutzhülle führt zu einer veränderten Dehnungs-/Druckcharakteristik des Druckausgleichsballons 1, sodass, nachdem der Ballon ein Volumen erreicht hat, bei welchem der Dehnungsabschnitt an den Wandabschnitten 5a anliegt, der Druck bei weiterer Gaszufuhr nicht mehr konstant bleibt, sondern kontinuierlich, aber wesentlich langsamer und besser kontrollierbar ansteigt als bei Verwendung einer kugelförmigen Schutzhülle 5. Auf diese Weise ist es möglich, in relativ kontrollierter Weise den Druck von dem normalen Regeldruck ausgehend, allmählich und kontinuierlich auf einen Druck zu erhöhen, wie er nach dem Ausfüllen der Ausbuchtungen 6 durch die sich da hinein ausdehnenden Teile des Dehnungsabschnittes 2' vorliegt. Dies ermöglicht eine deutlich erleichterte Einstellung eines bestimmten Druckwertes zwischen dem Regeldruck und dem erwähnten Enddruck, der beispielsweise in etwa das Doppelte des Regeldruckes betragen kann. Wenn also der Regeldruck im Bereich von 20 bis 25 mbar liegt, könnte der Enddruck zwischen 40 und 50 mbar liegen, wobei sich dieser Enddruck durch entsprechende Formgebung der Ausbuchtungen 6 und der dazwischen liegenden konvexen Abschnitte in größeren Bereichen varüeren lässt.

Ein entsprechend höherer Druck des Cuffs einer Beatmungskanüle ist beispielsweise erforderlich, um Blutungen zu stillen oder wenn bestimmte Behandlungen oder technische Maßnahmen am Patienten oder einer Beatmungskanüle zu treffen sind, die unvermeidlich mit stärkeren Bewegungen der Beatmungskanüle verbunden sind, wobei aber die Dichtigkeit des Cuffs in der Trachea nicht beeinträchtigt werden soll.

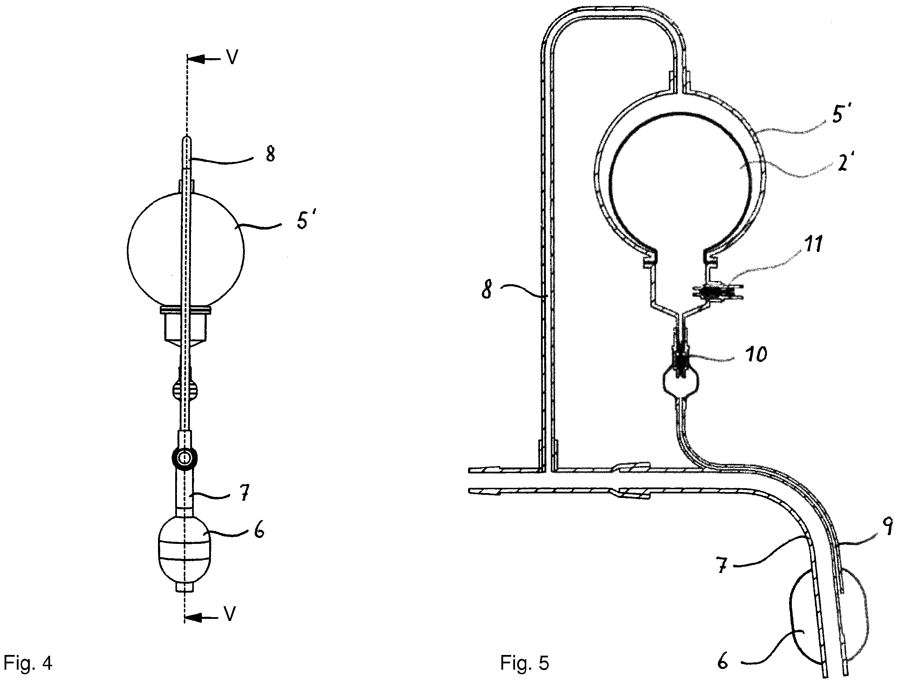

Figur 4 zeigt schematisch ein komplettes Beatmungssystem bestehend aus einer Beatmungskanüle 7 mit einem Cuff 6 und einem in einer kugelförmigen Schutzhülle 5' aufgenommenen Druckausgleichsballon. Die Details dieser Ausführungsform sind noch besser in der Schnittansicht gemäß Figur 5 zu erkennen. Figur 5 zeigt schematisch die Beatmungskanüle 7, den die Beatmungskanüle 7 umgebenden Cuff 6, der der Abdichtung des Zwischenraums zwischen der Wand der Trachea und der Außenseite der Kanüle 7 dient, mit einem Füllschlauch 9, der das Innere des Cuffs 6 mit einem Druckausgleichsballon 2' verbindet, wobei in diesem Fall zusätzlich noch ein Dämpfungsventil 10 in dem Füllschlauch 9 bzw. dessen Verbindung zu dem Druckausgleichsballon 2' vorgesehen ist, das dazu dient, den etwaigen Luft- bzw. Gasstrom aus dem Cuff 6 in den Ausgleichsballon 2' zu dämpfen. Bei der Beatmung und auch bei der Spontanatmung variiert der Druck in der Lunge und der Trachea des Patienten, wodurch wiederum von außen Druck auf den Cuff 6 ausgeübt wird, der dadurch Luft über den Füllschlauch 9 in den Ausgleichsballon 2' drückt. Dabei besteht die Gefahr, dass der Cuff 6 nicht mehr dicht an der Wand der Trachea anliegt.

Stattdessen sollte die Luft bzw. das Füllgas in dem Cuff 6 etwas länger verbleiben, wobei auch eine kurzzeitige Druckerhöhung in Kauf genommen wird, damit während einer Atemdruckspitze die Dichtigkeit der Anlage des Cuffs 6 an der Wand der Trachea gewährleistet bleibt. Das Dämpfungsventil 10 sorgt dafür, dass der Druck in dem Cuff 6 zumindest kurzzeitig, d.h. während entsprechender Atemdruckspitzen, höher sein kann als der Druck im Ausgleichsballon. In umgekehrter Richtung ist das Dämpfungsventil 10 jedoch durchlässig, sodass ein Nachlassen des Drucks im Cuff 6 unmittelbar ausgeglichen wird. Das Ventil 11 dient zur Befüllung des Cuffs und des Ausgleichsballons mit einer vorgegebenen Menge an Füllgas (zum Beispiel Luft oder Stickstoff).

Eine Alternative oder Ergänzung zu dem Dämpfungsventil 10 liegt bei dem erfindungsgemäßen Beatmungssystem in einem Verbindungsschlauch 8 zwischen der Beatmungskanüle 7 und der Schutzhülle 5', die bei dieser Ausführungsform auch kugelförmig ist bzw. sein kann.

Atemdruckspitzen, wie sie sowohl bei der künstlichen Beatmung als auch bei der Spontanatmung auftreten und die von außen auf den Cuff 6 wirken, liegen primär im Inneren der Beatmungskanüle 7 vor, sodass die entsprechenden Druckspitzen auch über den Verbindungsschlauch 8 in das Innere der Schutzhülle 5' übertragen werden und damit auch von außen auf den Ausgleichsballon 2' wirken. Durch die gleichzeitige Druckbeaufschlagung sowohl des Ausgleichsballons 2' als auch des Cuffs 6 von außen bleibt das System insgesamt besser im Gleichgewicht.

Allerdings bedeutet dies, dass der Innendruck in dem Cuff zusätzlich um den vollen Druck einer Atemluftspitze ansteigt. Falls also der Atemdruck 20 mbar beträgt und auch der Cuffdruck 20 mbar und der Atemdruck über den Verbindungsschlauch 8 in die Schutzhülle 5' übertragen wird, steigt der Druck im Druckausgleichsballon 2' ebenso wie in dem Cuff 6 um 20 mbar an, sodass der Cuffdruck insgesamt bereits 40 mbar beträgt, was auf Dauer unerwünscht sein könnte.

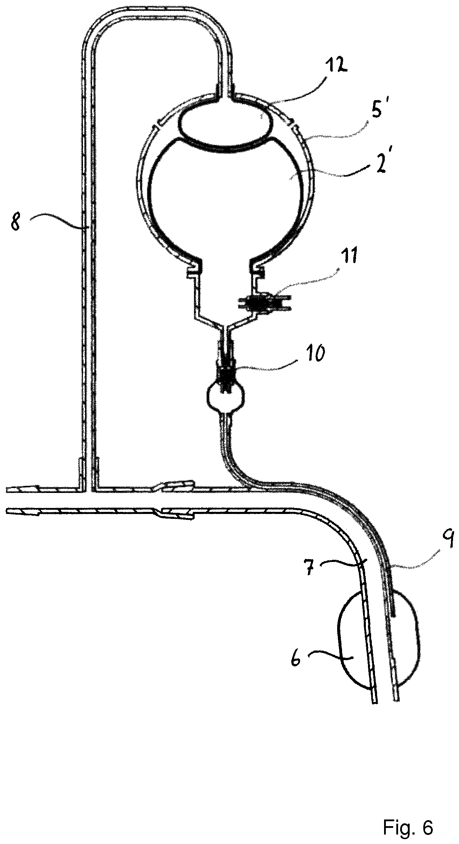

Um diesen Effekt etwas zu dämpfen, sieht die in Figur 6 dargestellte Ausführungsform noch eine Abwandlung dahingehend vor, dass nicht das gesamte Volumen der Schutzhülle 5' mit dem Atemdruck aus der Kanüle 7 beaufschlagt wird, sondern nur ein weiterer Übertragungsballon 12, der nur mit einem Teil der Oberfläche des Druckausgleichsballons 2' in Kontakt tritt, sodass der Druck im Inneren des Ausgleichsballons 2' nicht um den vollen Wert des Atemdrucks ansteigt.

Der Ballon kann aus einem dehnbaren Material bestehen, das analog zum Druckausgleichballon ausgeführt sein kann. Er kann jedoch auch aus einer dünnen Folie, die im typischen Druckbereich weitestgehend unelastisch ist bestehen (Luftsack). Der Luftsack kann in einer speziellen Ausführungsform kleine Löcher aufweisen, aus denen der Druck langsam entweichen kann. Dies führt dazu, dass der Cuffdruck aufgrund von abrupten Druckstößen lediglich kurzzeitig ansteigt und nicht dauerhaft.

Das Druckausgleichsprinzip gemäß der vorliegenden Erfindung kann auch für andere Produkte verwendet werden als für Tracheostomie- und Endotrachealkanülen, wie zum Beispiel für Larynxmasken. In diesem Fall müssen die Ballons lediglich etwas fester eingestellt werden, damit sie einen Solldruck von 50-70 mbar aufrecht erhalten.

Für Zwecke der ursprünglichen Offenbarung wird darauf hingewiesen, dass sämtliche Merkmale, wie sie sich aus der vorliegenden Beschreibung, den Zeichnungen und den abhängigen Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen und die Betonung der Unabhängigkeit der einzelnen Merkmale voneinander wird hier nur der Kürze und der Lesbarkeit der Beschreibung wegen verzichtet.

## Patentansprüche

1. Druckausgleichsballon, wobei das Volumen des Druckausgleichsballons mit einem weiteren Volumen in Verbindung steht, dessen Druck auch bei einer aufgezwungenen Volumenänderung einen möglichst konstanten Wert behalten soll, **dadurch gekennzeichnet, dass** der Ausgleichsballon aus einem thermoplastischen Elastomer (TPE), hergestellt ist und vor dem ersten Gebrauch über das maximal vorgesehene Regelvolumen hinaus überdehnt wurde und anschließend bis auf den Bereich des Regelvolumens relaxiert wurde, wobei das Überdehnungsvolumen mindestens das Doppelte des maximalen Regelvolumens beträgt, so dass der Elastizitätsmodul des TPE sich dahingehend verändert, dass der in dem Ausgleichsballon herrschende Druck mindestens innerhalb eines vorgegebenen Regelvolumenbereichs des Ausgleichsballons konstant bleibt, indem das Volumen des Ausgleichsballons bei einem über einen gewünschten Regelwert steigenden Druck zunimmt und bei einem unter den gewünschten Regelwert abnehmendem Druck abnimmt, wobei das Regelvolumen im Bereich von 20 cm³ bis 100 cm³ liegt.

2. Druckausgleichsballon nach Anspruch 1, **dadurch gekennzeichnet, dass** er aus einem styrolbasierten TPE, wie z. B. SBS (Styrol-Butadien-Styrol), SIS (Styrol-Isopren-Styrol), SEPS (Styrol-Ethylen-Propylen-Styrol) und insbesondere aus SEBS (Styrol-Ethylen-Butylen-Styrol) besteht und/oder dass der Ballon durch Spritzgießen hergestellt ist.

3. Druckausgleichsballon nach einem der vorstehenden Ansprüche, **dadurch gekennzeichnet, dass** die Wandstärke des drucklosen Ballons vor dem erstmaligen Überdehnen zwischen 0,4 und 1,5 mm liegt.

4. Druckausgleichsballon nach einem der vorstehenden Ansprüche, **dadurch gekennzeichnet, dass** der Ballon einen eine Ballonöffnung umgebenden verstärkten Öffnungsring aufweist, dessen Wandstärke mindestens 50% größer ist als die Wandstärke des Ballons im Übrigen.

5. Druckausgleichsballon nach einem der vorstehenden Ansprüche, **dadurch gekennzeichnet, dass** die Maße des Öffnungsrings an einen Standardanschluss mit einem Durchmesser zwischen 12 und 25 mm angepasst sind.

6. Druckausgleichsballon nach einem der vorstehenden Ansprüche, **dadurch gekennzeichnet, dass** das Regelvolumen den Bereich von 20 bis 80 cm³ umfasst.

7. Druckausgleichsballon nach einem der vorstehenden Ansprüche, **dadurch gekennzeichnet, dass** das Regelvolumen durch einen äußeren Schutzbehälter begrenzt ist.

8. Druckausgleichsballon nach einem der vorstehenden Ansprüche, **dadurch gekennzeichnet, dass** der zum erstmaligen Überdehnen erforderliche Druck mindestens 40 mbar beträgt.

9. Druckausgleichsballon nach einem der vorstehenden Ansprüche, **dadurch gekennzeichnet, dass** der Ballon auf einen Regelwert (Solldruckbereich) zwischen 20 und 30 mbar eingestellt ist.

10. Druckausgleichsballon nach einem der vorstehenden Ansprüche, **dadurch gekennzeichnet, dass** der Ballon abwechselnde, vorzugsweise streifenförmige Bereiche größerer und kleinerer Wandstärke aufweist.

11. Druckausgleichsballon nach einem der vorstehenden Ansprüche, **dadurch gekennzeichnet, dass** die Schutzhülle eine kugelförmige Grundform hat und/oder die Schutzhülle streifenförmige Ausbuchtungen aufweist.

12. Verfahren zur Herstellung eines Druckausgleichsballons nach einem der Ansprüche 1 - 11, **dadurch gekennzeichnet, dass** der Ballon aus einem TPE, vorzugsweise aus SEBS, im Spritzgussverfahren hergestellt und vor dem ersten Gebrauch auf ein Volumen überdehnt wird, welches größer als das im Gebrauch vorgesehene Regelvolumen ist, wobei das Überdehnungsvolumen mindestens das Doppelte des maximalen Regelvolumens beträgt, und anschließend wieder relaxiert wird.

13. Verfahren nach Anspruch 12, **dadurch gekennzeichnet, dass** eine Öffnung des Ballons mit einem die Öffnung umgebenden Öffnungsring größerer Wandstärke und definierter Geometrie für die Verbindung mit einem medizinischen Standardanschluss hergestellt wird.

## Claims

1. A pressure equalization balloon, wherein the volume of the pressure equalization balloon is connected to a further volume whose pressure is to maintain a value which is as constant as possible even in the event of a forced change in volume, **characterised in that** the equalization balloon is produced from a thermoplastic elastomer (TPE) and the balloon before first use was overstretched beyond the maximum intended regulating volume and is then relaxed to the range of the regulating volume, wherein the overstretching volume is at least 2 times the maximum regulating volume, so that the modulus of elasticity of the TPE changes in such a way that the pressure within the equalization balloon remains constant at least within a predetermined regulating volume range of the equalization balloon as the volume of the equalization balloon increases at a pressure rising above a desired regulating value and decreases at a pressure decreasing below the desired regulating value, wherein the regulating volume includes the range of from 20 cm³ to 100 cm³.

2. A pressure equalization balloon as set forth in claim 1 **characterised in that** it comprises a styrene-based TPE like for example SBS (styrene-butadiene-styrene), SIS (styreneisoprene-styrene), SEPS (styrene-ethylene-propylene-styrene) and in particular SEBS (styrene-ethylene-butylene-styrene) and/or that the balloon is produced by injection molding.

3. A pressure equalization balloon as set forth in one of the preceding claims **characterised in that** the wall thickness of the pressure-less balloon prior to the first-time overstretching is between 0.4 and 1.5 mm.

4. A pressure equalization balloon as set forth in one of the preceding claims **characterised in that** the balloon has a reinforced opening ring which extends around a balloon opening and whose wall thickness is at least 50% greater than the wall thickness of the balloon elsewhere.

5. A pressure equalization balloon as set forth in one of the preceding claims **characterised in that** the dimensions of the opening ring are matched to a standard connection of a diameter of between 12 and 25 mm.

6. A pressure equalization balloon as set forth in one of the preceding claims **characterised in that** the regulating volume includes the range of between 20 and 80 cm³.

7. A pressure equalization balloon as set forth in one of the preceding claims **characterised in that** the regulating volume is delimited by an outer protective container.

8. A pressure equalization balloon as set forth in one of the preceding claims **characterised in that** the pressure required for the first-time overstretching is at least 40 mbars.

9. A pressure equalization balloon as set forth in one of the preceding claims **characterised in that** the balloon is set to a regulating value (target pressure range) of between 20 and 30 mbars.

10. A pressure equalization balloon as set forth in one of the preceding claims **characterised in that** the balloon has alternate, preferably strip-shaped regions of larger and smaller wall thickness.

11. A pressure equalization balloon as set forth in one of the preceding claims **characterised in that** the protective case is of a spherical basic shape and/or the protective case has strip-shaped outward bulges.

12. A method for the production of a pressure equalization balloon as set forth in one of claims 1 through 11 **characterised in that** the balloon is produced from a TPE, preferably from SEBS, in an injection molding process and prior to the first use is overstretched to a volume greater than the regulating volume intended in use, wherein the overstretching volume is at least 2 times the volume of the maximum regulating volume, and is relaxed thereafter.

13. A method as set forth in claim 12 **characterised in that** an opening in the balloon is produced with an opening ring surrounding the opening and of larger wall thickness and of defined geometry for connection to a standard medical connector.

## Revendications

1. Ballonnet de compensation de pression, le volume du ballonnet de compensation de pression communiquant avec un autre volume dont la pression est censée garder une valeur la plus constante possible, même lors d'un changement forcé de volume, **caractérisé en ce que** le ballonnet de compensation est fabriqué à partir d'un élastomère thermoplastique (TPE) et a été distendu, avant la première utilisation, au-delà du volume régulier maximal prévu et a été relâché ensuite jusqu'à la plage du volume régulier, le volume de distension étant au moins le double du volume régulier maximal, si bien que le module d'élasticité du TPE soit modifié avec le but que la pression existant dans le ballonnet de compensation reste constante au moins dans une plage prédéterminée de volumes réguliers, par le fait que le volume du ballonnet de compensation augmente lorsque la pression dépasse une valeur régulière souhaitée et diminue lorsque la pression passe en dessous de la valeur régulière souhaitée, le volume régulier étant dans la plage de 20 cm³ à 100 cm³.

2. Ballonnet de compensation de pression selon la revendication 1, **caractérisé en ce qu'**il est fabriqué à partir d'un TPE à base de styrol tel que par exemple SBS (styrol-butadiène-styrol), SIS (styrol-isoprène-styrol), SEPS (styrol-éthylène-propylène-styrol) et notamment SEBS (styrol-éthylène-butylène-styrol) et/ou que le ballonnet a été fabriqué par moulage par injection.

3. Ballonnet de compensation de pression selon l'une des revendications précédentes, **caractérisé en ce que** l'épaisseur de paroi du ballonnet hors pression avant la première distension est entre 0,4 et 1,5 mm.

4. Ballonnet de compensation de pression selon l'une des revendications précédentes, **caractérisé en ce que** le ballonnet comprend une bague d'ouverture renforcée entourant une ouverture de ballonnet, l'épaisseur de paroi de la bague étant d'au moins 50 % supérieure à l'épaisseur du reste du ballonnet.

5. Ballonnet de compensation de pression selon l'une des revendications précédentes, **caractérisé en ce que** les dimensions de la bague d'ouverture sont adaptées à un raccord standard ayant un diamètre entre 12 et 25 mm.

6. Ballonnet de compensation de pression selon l'une des revendications précédentes, **caractérisé en ce que** le volume régulier comprend la plage de 20 à 80 cm³.

7. Ballonnet de compensation de pression selon l'une des revendications précédentes, **caractérisé en ce que** le volume régulier est limité par un contenant protecteur extérieur.

8. Ballonnet de compensation de pression selon l'une des revendications précédentes, **caractérisé en ce que** la pression nécessaire pour une première distension est d'au moins 40 mbar.

9. Ballonnet de compensation de pression selon l'une des revendications précédentes, **caractérisé en ce que** le ballonnet est ajusté à une valeur régulière (plage de pressions de consigne) entre 20 et 30 mbar.

10. Ballonnet de compensation de pression selon l'une des revendications précédentes, **caractérisé en ce que** le ballonnet comprend des zones alternantes, de préférence en forme de bandes, d'épaisseurs de paroi supérieure et inférieure.

11. Ballonnet de compensation de pression selon l'une des revendications précédentes, **caractérisé en ce que** le contenant protecteur présente une forme de base en forme de sphère et/ou que le contenant protecteur comprend des convexités en forme de bandes.

12. Procédé de fabrication d'un ballonnet de compensation de pression selon l'une des revendications 1 à 11, **caractérisé en ce que** le ballonnet est fabriqué à partir d'un TPE, de préférence en SEBS, par un procédé de moulage par injection et est distendu, avant la première utilisation, à un volume qui est supérieur au volume régulier prévu pour l'utilisation, le volume de distension étant au moins le double du volume régulier maximal, et est relâché ensuite.

13. Procédé selon la revendication 12, **caractérisé en ce qu'**il est réalisé une ouverture du ballonnet avec une bague d'ouverture entourant l'ouverture et ayant une épaisseur de paroi supérieure et une géométrie définie pour un raccordement à un raccord standard médical.
